# NEW EUROPEAN PATENT SPECIFICATION

(11) **EP 2 164 943 B2**
(45) Date of publication and mention of the opposition decision: **20.09.2017**
(45) Mention of the grant of the patent: 12.03.2014
(21) Application number: 08760676.0
(22) Date of filing: 06.06.2008
(51) Int. Cl.: C11D 3/386, D06M 16/00, C12N 9/08, C11D 3/00, C12N 9/42

(54) **A PROCESS FOR COMBINED BIOPOLISHING AND BLEACH CLEAN-UP**
VERFAHREN FÜR KOMBINIERTE BIOPOLIERUNG UND BLEICHREINIGUNG
PROCEDE POUR BIOPOLISSAGE ET NETTOYAGE PAR UN AGENT DE BLANCHIMENT COMBINES

(30) Priority: 11.06.2007 EP 07109969
(43) Date of publication of application: 24.03.2010
(60) Divisional application: 13196997.4
(73) Proprietor: Novozymes A/S, 2880 Bagsvaerd (DK)
(72) Inventor: KUILDERD, Harm, Albertus, Beijing 100103 (CN); WU, Guifang, Beijing 100085 (CN); LI, Haijing, Beijing 100085 (CN); ZHOU, Quan, Beijing 100085 (CN)
(74) Representative: Potter Clarkson LLP
(86) International application number: PCT/EP2008/057106
(87) International publication number: WO 2008/151999

(56) References cited:
- WO-A-00/71808
- WO-A-99/32708
- WO-A-03/002810
- WO-A1-02/38717
- WO-A1-91/17243
- WO-A1-91/17243
- WO-A1-92/17571
- WO-A1-93/17721
- WO-A1-96/29397
- WO-A1-96/29397
- WO-A1-2004/059075
- WO-A1-2004/059075
- WO-A1-2014/026630
- WO-A2-2006/101584
- WO-A2-2006/101584
- WO-A2-2007/019442
- WO-A2-2007/019442
- WO-A2-2008/023386
- CN-A- 1 584 192
- CN-A- 1 584 192
- GB-A- 2 216 149
- PT-A- 101 152
- US-A- 5 700 769
- US-A- 6 140 109
- ROY S K, DEY S K, RAHA S K, CHAKRABARTY S L: "Purification and properties of an extracellular endoglucanase from Myceliophthora thermophila", JOURNAL OF GENERAL MICROBIOLOGY, vol. 136, 1 January 1990 (1990-01-01), pages 1967-1971,
- SHACTER E: "Quantification and significance of protein oxidation in biological samples", DRUG METABOLISM REVIEWS, no. 32(3&4) , pages 307-326,
- ANKENY, M: "Single-bath Dyeing and Bio-polishing", AATCC REVIEW, vol. 2, 1 May 2002 (2002-05-01), pages 16-19,
- "Neutral Bio-polishing gives even better fabric qulaity", Biotimes, 02/06
- Product Application Guide Novozymes - Cellusoft CR
- IAN HOLME: "Pretreatment Enzymes for Combined Treatments", INTERNATIONAL DYER, 1 February 2003 (2003-02-01), pages 9-11,
- 'PRODUCT APPLICATION GUIDELINE' NOVOZYMES CELLUSOFT CR 2006,
- 'NEUTRAL BIO-POLISHING GIVES EVEN BETTER FABRIC QUALITY' BIOTIMES, [Online] 01 February 2006, pages 4 - 5, XP003028277 Retrieved from the Internet: <URL:HTTP://WWW.BIOTIMES.COM/EN/ARTICLES/20 06/JUNE/DOCUMENTS/JUNE%202006_BIO-POLISHING _GB.PDF> [retrieved on 2011-11-01]
- HOLME I.: 'PRETREATMENT ENZYMES FOR COMBINED TREATMENTS' INTERNATIONAL DYER vol. 188, no. 2, 01 February 2003, WORLD TEXTILE PUBLICATIONS, BRADFORD, GB, pages 9 - 11, XP001233976
- ANKENY M.: 'SINGLE-BATH DYEING AND BIO-POLISHING' AATCC REVIEW vol. 2, 01 May 2002, pages 16 - 19, XP003028279
- PEDERSEN ET AL: 'BIOPOLISHING OF CELLULOSIC FABRICS' CANADIAN TEXTILE JOURNAL December 1992, pages 31 - 35
- PRODUCT SHEET FOR CELLUSOFT AP (NOVO NORDISK A/S) 1999
- PRODUCTSHEET FOR CELLUZYME (NOVO INDUSTRI A/S) 1987
- SCHÜLEIN M.: 'KINTICS OF FUNGAL CELLULASES' BIOCHEMICAL SOCIETY TRANSACTIONS vol. 26, 1998,

## Description

### Reference to a Sequence Listing

This application contains a Sequence Listing in computer readable form. The computer readable form is incorporated herein by reference.

### Field of the Invention

The present invention relates to methods for treating textile, and more particularly, to methods for one-step combined biopolishing and bleach clean-up, comprising treating textile with a system for removing hydrogen peroxide and an enzyme system for bio-polishing.

### Background of the Invention

Usually, there is a process to remove the residual hydrogen peroxide after Bleaching. There are three kinds of way to do it. One is washing with water several times, one is treating the bath with reducing agents and the other is with the use of catalase. The using of catalase has become more and more popular. The preparation process, which may involve desizing (for woven goods), scouring, bleaching and bleach clean up, produces a textile suitable for dyeing. Bio-polishing is done before or after dyeing. Up to now, the Bleach Clean up, BioPolishing and dyeing are done in separate steps, see Int. Dyer 188 : 9 - 11 (2003), Pretreatment enzymes for combined treatments, Holme I.

A. BioPolishing: Bio-Polishing is a specific treatment of the yarn surface which improves fabric quality with respect to handle and appearance. The most important effects of BioPolishing can be characterized by less fuzz and pilling, increased gloss/luster, improved fabric handle, increased durable softness and improved water absorbency. Bio-Polishing usually takes place in the wet processing of the manufacture of knitted and woven fabrics. Wet processing comprises such steps as e.g. desizing, scouring, bleaching, washing, dying/printing and finishing.

B. Bleach Clean-up: The purpose of bleaching is to completely remove colored impurities, improve absorbency, and achieve adequate whiteness and dyeability. In order to obtain clear, pure tones on a variety of fibres (particularly on natural fibres), it is necessary to bleach them prior to dyeing. This enables one to obtain a good ground fibre. From an ecological and technical point of view, hydrogen peroxide, its derivatives and addition products that release peroxide are generally used for the bleaching process. However, generally, excess peroxide product remains on the fibre and when this occurs it can interfere with and have an adverse affect on subsequent dyeings with anionic dyes, for example, reactive dyes where the dye is in part or totally destroyed.

Destroying excess H₂O₂ after bleaching is referred to as the Bleach Clean-up process. This can be done by applying a catalase to the substrate. In order to obtain consistent, high quality results with commercial quantities of textiles, the Bleach Clean-up or biopolishing steps are usually performed separately because it is very difficult to combine the enzymatic processes due to processing constraints related to pH.

C. Dyeing: Dyeing of textiles is often considered to be the most important and expensive single step in the manufacturing of textile fabrics and garments. The major classes of dyes are azo (mono-, di-, tri-, etc.), carbonyl (anthraquinone and indigo derivatives), cyanine, di- and triphenylmethane and phthalocyanine. All these dyes contain chromophore groups, which give rise to color. These chemical structures constitute several cellulosic dye classes, i.e. vat, sulfur, azoic, direct, and reactive dyes as defined in the Colour Index. Three of these dye types involve an oxidation/reduction mechanism, i.e., vat, sulfur and azoic dyes. The purpose of the oxidation/reduction step in these dyeing procedures is to change the dyestuff between an insoluble and a soluble form.

Processing and dyeing procedures are performed in either a batch or continuous mode, with the fabric being contacted by the liquid processing stream in open width or rope form. In continuous methods, a saturator is used to apply chemicals to the fabric, after which the fabric is heated In a chamber where the chemical reaction takes place. A washing section then prepares the fabric for the next processing step. Batch processing generally takes place in one processing bath whereby the fabric is circulated through the bath. After a reaction period, the chemicals are drained, fabric rinsed and the next chemical is applied. Discontinuous pad-batch processing Involves a continuous application of processing chemical followed by a dwell period, which, in the case of cold pad-batch, might be one or more days.

Regardless of whether batch, continuous, or discontinuous pad-batch methods are used, bleach clean up, biopolishing and dyeing steps have not heretofore been compatible, due to the broad variation of conditions present in each of the steps. Consequently, it has been necessary to rinse or otherwise treat the fabric or to replace the treating solutions between bleach clean up, biopolishing and dyeing. Thus, there is a need in the art for harmonization of these three steps so that they can be performed in a single bath simultaneously, so as to shorten processing time, conserve materials, and reduce the waste stream.

### Summary of the Invention

Therefore, a first aspect of the present invention provides a one-step textile treatment process for combined biopolishing and hydrogen peroxide beach clean up, as defined in Claim 1.

The process of biopolishing and beach clean up can be further combined with dyeing step and performed in a single solution. Accordingly, in one embodiment of the first aspect of the present invention, the solution is supplemented with one or more dyestuffs, and the method comprises incubating the textile for a sufficient time to achieve dyed textile.

Preferably, the dyestuff is reactive dye. Preferably, the system for removing hydrogen peroxide is the enzyme system comprises catalase. The enzyme system for bio-polishing comprises celluase and may further comprise protease.

In an alternative embodiment, the system for removing hydrogen peroxide is chemical reducing agent. The enzyme system for bio-polishing comprises cellulase, and may further comprise protease.

Preferably, the textile is cellulose-containing or cellulosic fabrics, such as cotton, viscose, rayon, ramie, linen, lyocell, or mixtures thereof, or mixtures of any of these fibers together with synthetic fibres or other natural fibers.

Preferably, the cellulase is derived from species of *Humicola, Thermomyces, Bacillus, Trichoderma, Fusarium, Myceliophthora, Phanerochaete, Irpex, Scytalidium, Schizophyllum, Penicillium, Thielavia, Aspergillus,* or *Geotricum, and more preferably* the cellulase is derived from *Thielavia terrestis, Myceliophthora thermophila, Humicola insolens, Trichoderma reesel, Trichoderma harzianum, Aspergillus aculeatus,* and *Aspergillus niger.*

Preferably, the catalase is derived from bacteria such as *Bacillus, Pseudomonas or Streptomyces strain;* yeast such as *Candida, Kluyveromyces, Pichia, Saccharomyces, Yarrowia or Schizosaccharomyces*; fungal such as *Acremonium, Aspergillus, Coprinus, Auraobasidium, Bjerkandera, Humicola, Ceriporiopsis, Coriolus, Cryptococcus, Filibasidium, Fusarium, Magnaporthe, Mucor, Myceliphthora, Neocallimastix, Neurospora, Paecilomyces, Penicillium, Phanerochaete, Phlebia, Piromyces, Pleurotus, Schizophyllum, Talaromyces, Thermoascus, Thielavia, Tolypocladium, Trametes or Trichoderma strain;* or animal such as pig liver, beef lever.

Accordingly, an enzyme composition suitable for use in the first aspect of the present invention comprises (i) a catalase in combination with (ii) an enzyme system comprising cellulase for bio-polishing.

When biopolishing and bleach clean up take place in the same treating solution, compared to traditional processes involving separated biopolishing and bleach clean up, a pH adjusting step and temperature adjusting step are avoided in the present invention. Another advantage of the invention is that process time is saved/reduced as biopolishing and bleach clean up may be carried out simultaneously. The methods of the present invention of combining bio-polishing and bleaching clean-up in one step thus save time, energy and water. Furthemore, when dyeing step is combined with bleach clean up and biopolishing in the same treating solution, it make the process even more efficient.

### Detailed Description of the Invention

### Textiles

In context of the invention the term "textile" referes to fabrics.

Fabric can be constructed from fibers by weaving, knitting or non-woven operations. Weaving and knitting require yarn as the input whereas the non-woven fabric is the result of random bonding of fibers (paper can be thought of as non-woven). In the present context, the term "fabric" is also intended to include fibers and other types of processed fabrics.

Woven fabric is constructed by weaving "filling" or weft yarns between wrap yarns stretched in the longitudinal direction on the loom. The wrap yarns must be sized before weaving in order to lubricate and protect them from abrasion at the high speed insertion of the filling yarns during weaving. The filling yarn can be woven through the warp yarns in a "over one - under the next" fashion (plain weave) or by "over one - under two" (twill) or any other myriad of permutations. Strength, texture and pattern are related not only to the type/quality of the yarn but also the type of weave. Generally, dresses, shirts, pants, sheeting's, towels, draperies, etc. are produced from woven fabric.

Knitting is forming a fabric by joining together interlocking loops of yam. As opposed to weaving which is constructed from two types of yarn and has many "ends", knitted fabric is produced from a single continuous strand of yam. As with weaving, there are many different ways to loop yarn together and the final fabric properties are dependent both upon the yarn and the type of knit. Underwear, sweaters, socks, sport shirts, sweat shirts, etc. are derived from knit fabrics.

Non-woven fabrics are sheets of fabric made by bonding and/or interlodcing fibers and filaments by mechanical, thermal, chemical or solvent mediated processes. The resultant fabric can be in the form of web-like structures, laminates or films. Typical examples are towels, wipes, surgical gowns, fibers for the "environmental friendly" fashion, filter media, bedding, roofing materials, backing for two-dimensional fabrics and many others.

According to the invention, the method of the invention may be applied to any fabric known in the art (woven, knitted, or non-woven). In particular the process of the present invention may be applied to cellulose-containing or cellulosic fabrics, such as cotton, viscose, rayon, ramie, linen, lyocell (e.g. Tencel, produced by Courtaulds Fibers), or mixtures thereof, or mixtures of any of these fibers together with synthetic fibres (e.g., polyester, polyamid, nylon) or other natural fibers such as wool and silk., such as viscose/cotton blends, lyocell/cotton blends, viscose/wool blends, lyocell/wool blends, cotton/wool blends; flax (linen), ramie and other fabrics based on cellulose fibers, including all blends of cellulosic fibers with other fibers such as wool, polyamide, acrylic and polyester fibers, e.g., viscose/cotton/polyester blends, wool/cotton/polyester blends, flax/cotton blends etc. The term "wool," means any commercially useful animal hair product, for example, wool from sheep, camel, rabbit, goat, llama, and known as merino wool, Shetland wool, cashmere wool, alpaca wool, mohair, etc. and includes wool fiber and animal hair. The method of the invention can be used with wool or animal hair material in the form of top, fiber, yam, or woven or knitted fabric. The enzymatic treatment can also be carried out on loose flock or on fibers made from wool or animal hair material. The treatment can be performed at many different stages of processing. The fabric to be bleached may be dyed or undyed. According to the invention textile may be desized, scoured and/or bleached in aqueous medium in the presence of a fatty acid oxidizing enzyme.

### Enzyme composition

As used in the present invention, an enzyme composition comprises a bio-polishing enzymes system comprising cellulase, and a hydrogen peroxide removing system comprising a catalase.

"Bio-polishing enzymes system" comprises one or more bio-polishing enzyme(s), at least one of which is a cellullase. Other suitable enzymes for further inclusion in a "bio-polishing enzymes system" include protease. "Hydrogen peroxide removing system" comprises catalase.

### Blo-polishing Enzymes:

The process of the invention comprises cellulase (and optionally also protease) treatment of the fabric.

Cellulase is generally used in biopolishing process for textile like cellulosic fabrics, and cellulosic fabrics/synthetic fibres blends, while protease is used in shrinkage-resistance process for natural protein fibers such as wool and silk. Preferably, cellulase together with protease can be used in biopolishing process of blends of cellulosic fibers with other fibers, such as cellulosic fabrics/wool blends, cellulosic fabrics/synthetic fibres/wool blends and etc.

### Cellulase

The term "cellulase" denotes an enzyme that contributes to the hydrolysis of cellulose, such a cellobiohydrolase (abbreviated as "CBH", Enzyme Nomenclature E.C. 3.2.1.91), an endoglucanase (hereinafter abbreviated as "EG", E.C. 3.2.1.4), or a beta-glucosidase (abbreviated as "BG", E.C. 3.2.1.21). Cellulases are classified in a series of enzyme families encompassing endo- and exo- activities as well as cellobiose hydrolyzing capability. The cellulase used in practicing the present invention may be derived from microorganisms which are known to be capable of producing cellulolytic enzymes, such as, e.g., species of *Humicola, Thermomyces, Bacillus, Trichoderma, Fusarium, Myceliophthora, Phanerochaete, Irpex, Scytalidium, Schizophyllum, Penicillium, Thielavia, Aspergillus,* or *Geotricum,* particularly *Humicola insolens, Fusarium oxysporum*, or *Trichoderma reesei.* Non-limiting examples of suitable cellulases are disclosed in U.S. Patent No. 4,435,307; European patent application No. 0 495 257; PCT Patent Application No. WO91/17244, WO91/17243, WO98/12307; and European Patent Application No. EP-A2-271 004.

The cellulases used in this invention can be monocomponent or multi-components. Monocomponent, i.e. a cellulase which is essentially free from other proteins, in particular other cellulases. Monocomponent enzymes can be prepared economically by recombinant DNA technology, i.e. they can be produced by cloning of a DNA sequence encoding the monocomponent, subsequently transforming a suitable host cell with the DNA sequence and expressing the component in the host. Cellulases of multi-components contain more than one cellulase. Cellulases of muti-components can be blends of two or more cellulases, or can be derived from wild type strain.

The DNA sequence coding for a useful cellulase may for instance be isolated by screening a cDNA library of the microorganism in question and selecting for clones expressing the appropriate enzyme activity (i.e. cellulase activity).

A DNA sequence coding for a homologous enzyme, i.e. an analogous DNA sequence, may be obtainable from other microorganisms. For instance, the DNA sequence may be derived by similarly screening a cDNA library of another fungus, such as a strain of an *Aspergillus sp.,* in particular a strain of *A. aculeatus* or *A. niger,* a strain of *Trichoderma sp.,* in particular a strain of *T. reesei, T. viride, T. longibrachiatum, T. harzianum* or *T. koningii* or a strain of a *Neocallimastix sp., a Piromyces sp., a Penicillium sp.,* an *Agaricus sp.,* or a *Phanerochaete sp.*

Preferably, the cellulase is derived from or producible by a strain of *Scytalidium (f. Humicola), Fusarium, Myceliophthora,* more preferably derived from or producible by *Scytalidium thermophilum (f. Humicola insolens), Fusarium oxysporum or Myceliophthora themophilia,* most preferably from *Humicola insolens, DSM 1800, Fusarium oxysporum, DSM 2672*, or Myceliophthora *themophila, CBS117.65.*

In one embodiment of the invention, the cellulase is an endoglucanase, preferably cellulase from family 45 EG, such as the amino acid sequence of the Thielavia terrestis endoglucanase shown in SEQ ID No. 1 (as described in WO 96/29397) or is an analogue of said endoglucanase which is at least 60% homologous with the sequence shown in SEQ ID No. 1, reacts with an antibody raised against said endoglucanase, and/or is encoded by a DNA sequence which hybridizes with the DNA sequence encoding said endoglucanase. In another embodiment of the invention, the cellulase is an endoglucanase comprising the amino acid sequence of the Humicola insolens endoglucanase shown in SEQ ID No. 2 (as described in WO 91/17243) or is an analogue of said endoglucanase which is at least 60% homologous with the sequence shown in SEQ ID No. 2, reacts with an antibody raised against said endoglucanase, and/or is encoded by a DNA sequence which hybridizes with the DNA sequence encoding said endoglucanase. In a further embodiment of the invention, the celllulases used in the invention are commercially available multi-components cellulase enzyme product such as Cellusoft L, Cellish L (Novozymes A/S, Denmark), Primafast 100, Primafast 200 (Genencor International Inc.), Rocksoft ACE (Dyadic), and Youteer 800□(Youteer Co. Ltd, China).

The host cell which is transformed with the DNA sequence is preferably a eukaryotic cell, in particular a fungal cell such as a yeast or filamentous fungal cell. In particular, the cell may belong to a species of *Aspergillus* or *Trichoderma,* most preferably *Aspergillus* oryzae or *Aspergillus niger*. Fungal cells may be transformed by a process involving protoplast formation and transformation of the protoplast followed by regeneration of the cell wall in a manner known per se The use of *Aspergillus* as a host microorganism is described in EP 238 023 (Novo Nordisk A/S), the contents of which are hereby incorporated by reference. The host cell may also be a yeast cell, e.g. a strain of *Saccharomyces,* In particular *Saccharomyces* cerevisiae, *Saccharomyces kluyveri* or *Saccharomyces uvarum,* a strain of *Schizosaccharomyces sp*., such as *Schizosaccharomyces pombe,* a strain of *Hansenula sp., Pichia sp., Yarrowia sp.* such as *Yarrowia lipolytica,* or *Kluyveromyces sp.* such as *Kluyveromyces lactis*.

In the context, an analogue of the proteins comprises "variant proteins". In some preferred embodiments, variants proteins differ from a parent protein, e.g., a wild-type protein, and one another by a small number of amino acid residues. In the present context, the term "homologous" or "homologous sequence" is intended to indicate an amino acid sequence differing from another protein, by one or more amino acid residues, preferably 1, 2, 3, 4, 5, 10, 15, 20, 30, 40, 50 or more amino acid residues. For example, in some embodiments, variant proteins have one to ten difference from the parent protein. The homologous sequence may be one resulting from modification of an amino acid sequence shown in these listings, e.g. involving substitution of one or more amino acid residues at one or more different sites in the amino acid sequence, deletion of one or more amino acid residues at either or both ends of the enzyme or at one or more sites in the amino acid sequence, or insertion of one or more amino acid residues at one or more sites in the amino acid sequence.

However, as will be apparent to the skilled person, amino acid changes are preferably of a minor nature, that is conservative amino acid substitutions that do not significantly affect the folding or activity of the protein, small deletions, topically of one to about 30 amino acids; small amino- or carboxyl-terminal extensions, such as an amino-terminal methionine residue, a small linker peptide of up to about 20-25 residues, or a small extension that facilitates purification, such as a poly-histidine tract, an antigenic epitope or a binding domain. See in general Ford et al., Protein Expression and Purification 2: 95-107, 1991. Examples of conservative substitutions are within the group of basic amino acids (such as arginine, lysine, histidine), acidic amino acids (such as glutamic acid and aspartic acid), polar amino acids (such as glutamine and asparagine), hydrophobic amino acids (such as leucine, isoleucine, valine), aromatic amino acids (such as phenylalanine, tryptophan, tyrosine) and small amino acids (such as glycine, alanine, serine, threonine, methionine).

The modification of the amino acid sequence may suitably be performed by modifying the DNA sequence encoding the enzyme, e.g. by site-directed or by random mutagenesis or a combination of these techniques in accordance with well-known procedures. Alternatively, the homologous sequence may be one of an enzyme derived from another origin than the cellulases corresponding to the amino acid sequences shown in each of the sequence listings shown hereinafter, respectively. Thus, "homologue" may e.g. indicate a polypeptide encoded by DNA which hybridizes to the same probe as the DNA coding for the cellulase with the amino acid sequence in question under certain specified conditions (such as presoaking in 5 x SSC and prehybridising for 1 h at ∼40°C in a solution of 20% formamide, 5 x Denhardt's solution, 50 mM sodium phosphate, pH 6.8, and 50 mg of denatured sonicated calf thymus DNA, followed by hybridization in the same solution supplemented with 100 mM ATP for 18 h at ∼40°C). The homologous sequence will normally exhibit a degree of homology (in terms of identity) of at least 50%, such as at least 60%, 65%, 70%, 75%, 80%, 85%, 90% or even 95% with the amino acid sequences shown in each of the sequence listings shown hereinafter, respectively.

The homology referred to above is determined as the degree of identity between the two sequences indicating a derivation of the first sequence from the second. The homology may suitably be determined by means of computer programs known in the art such as GAP provided in the GCG program package (Needleman, S.B. and Wunsch, C.D., Journal of Molecular Biology, 48: 443-453, 1970).

### Proteases

Any protease suitable for use in the present invention may be employed.

Suitable proteases include those of animal, vegetable or microbial origin, preferably of microbial origin. Preferably, the protease may be a serine protease or a metalloprotease, more preferably, an alkaline microbial protease or a trypsin-like protease. Examples of proteases include aminopeptidases, including prolyl aminopeptidase (3.4.11.5), X-pro aminopeptidase (3.4.11.9), bacterial leucyl aminopeptidase (3.4.11.10), thermophilic aminopeptidase (3.4.11.12), lysyl aminopeptidase (3.4.11.15), tryptophanyl aminopeptidase (3.4.11.17), and methionyl aminopeptidase (3.4.11.18); serine endopeptidases, including chymotrypsin (3.4.21.1), trypsin (3.4.21.4), cucumisin (3.4.21,25), brachyurin (3.4.21.32), cerevisin (3.4.21.48) and subtilisin (3.4.21.62); cysteine endopeptidases, including papain (3.4.22.2), ficain (3.4.22.3), chymopapain (3.4.22.6), asclepain (3.4.22.7), actinidain (3.4.22.14), caricain (3.4.22.30) and ananain (3.4.22.31); aspartic endopeptidases, including pepsin A (3.4.23.1), Aspergillopepsin I (3.4.23.18), Penicillopepsin (3.4.23.20) and Saccharopepsin (3.4.23.25); and metalloendopeptidases, including Bacillolysin (3.4.24.28).

Commercially available proteases include Alcalase, Savinase, Primase, Duralase, Esperase, Kannase, and Durazym (available from Novozymes A/S), Maxatase, Maxacal, Maxapem, Properase, Purafect, Purafect OxP, FN2, FN3 and FN4 (available from Genencor International Inc.).

Also useful in the present invention are protease variants, such as those disclosed in EP 130,756 (Genentech), EP 214,435 (Henkel), WO 87/04461 (Amgen), WO 87/05050 (Genex), EP 251.446 (Genencor), EP 260.105 (Genencor), Thomas et al., (1985), Nature. 318, p. 375-376, Thomas et al., (1987), J. Mol. Biol., 193, pp. 803-813, Russel et al., (1987), Nature, 328, p. 496-500, WO 88/08028 (Genex), WO 88/08033 (Amgen), WO 89/06279 (Novozymes A/S), WO 91/00345 (Novozymes A/S), EP 525 610 (Solvay) and WO 94/02618 (Gist-Brocades N.V.). The activity of proteases can be determined as described in "Methods of Enzymatic Analysis", third edition, 1984, Verlag Chemie, Weinheim, vol. 5.

### Bleach clean-up system:

The Bleach Clean-up system is a catalase as a Bleach Clean-up enzyme.

### Bleach clean-up Enzymes:

Bleach clean-up enzymes refer to catalase enzymes which can catalyze for the conversion of hydrogen peroxide into water and oxygen. Preferred catalases that are suitable for use in a process according to the invention are catalases that is derived from bacteria such as *Bacillus, Pseudomonas or Streptomyces strain;* yeast such as *Candida, Kluyveromyces, Pichia, Saccharomyces, Yarrowia or Schizosaccharomyces;* fungal such as *Acremonium, Scytalidium, Aspergillus, Coprinus, Aureobasidium, Bjerkandera, Humicola, Ceriporiopsis, Coriolus,* Cryptococcus, *Filibasidium, Fusarium, Magnaporthe, Mucor, Myceliphthora, Neocallimastix, Neurospora, Paecilomyces, Penicillium, Phanerochaete, Phlebia, Piromyces, Pleurotus, Schizophylium, Talaromyces, Thermoascus, Thielavia, Tolypocladium, Trametes or Trichoderma strain;* or animal such as pig liver, beef lever.. Non-limiting examples of suitable catalases are disclosed in WO92/17571, CN1563373, US2003100112-A1, EP1336659-A, US2003074697-A1, US6201167-91, US6022721-A, EP931831-A, JP11046760-A, WO9317721-A, WO9309219-A, JP1086879-A and/or JP63003788-A. Non-limiting examples are T 100; Oxy-Gone 400 (GCI); Fermcolase 1000 (Mitsubishi Gas Chemical) or Thermocatalase CTL 200 or JH CT 1800 (Mitsubishi Gas Chemical).

In one embodiment of the present invention, the catalase used is derived from *Scytalidium thermophilum* with SEQ ID No:3 (as described in US 5,646,025), or analogue or particularly variant proteins of it.

Depending on the activity of the catalase and the pH of the liquor used to apply the catalase, preferably the amount of catalase used is from 0.001 to 1 g/l, especially about 5 g/l of liquor used to apply the catalase.

### Dyestuff

In practicing the present invention, any dyestuff may be used that is compatible with the conditions used for biopolishing.

Conventional dyeing stuffs, comprising one or more of reactive dyes, such as, e.g., C. I. Reactive Red 1, 3, 6, 17, 120, 194, Blue 4, 19, 171 and 182, Black 5, Violet 5; vat dyes, such as, e.g., C. I. Vat Yellow 28, Orange 11 and 15, Blue 6, 16 and 20; Green 1 and 3, 8, Brown 1, Black 9, 27; direct dyes, such as, C. I. Direct Red 81, Yellow 11 and 28, Orange 39, Red 76, Blue 78, 86, 106, 107 and 108, Black 22; sulfur dyes, such as, e.g., C. I. Sulfur Black 1 and 11, Brown 1, Red 10; and azoic dyes, such as, e.g., C. I. Coupling Components 5 and 13 in combination with C. I. Azoic Diazo Components 44 and 45. Such dyes are well known in the art and are described, e.g., in Shore, ed., Cellulosic Dyeing, Society of Dyers and Colorists, Alden Press, 1995; and in Colour Index, Society of Dyers and Colorists and American Association of Textile Chemists and Colorists, Vols. 1-8 Supplements, 1977-1988.

### Additional components:

In some embodiments of the invention, the aqueous solution or wash liquor further comprises other components, including without limitation other enzymes, as well as surfactants, stabilizer, wetting agent, dispersing agents, antifoaming agents, lubricants, builder systems, and the like, or a mixture thereof, that enhance the scouring and/or bleaching processes and/or provide superior effects related to, e.g., strength, resistance to pilling, water absorbency, and dyeability.

The enzymes may be isolated from their cell of origin or may be recombinantly produced, and may be chemically or genetically modified. It will be understood that the amount of enzymatic activity units for each additional enzyme to be used in the methods of the present invention in conjunction with a particular bio-polishing enzyme can be easily determined using conventional assays.

Surfactants suitable for use in practicing the present invention include, without limitation, nonionic, anionic; cationic; and zwitterionic surfactants; which are typically present at a concentration of between about 0.2% to about 15% by weight, preferably from about 1% to about 10% by weight. Anionic surfactants include, without limitation, linear alkylbenzenesulfonate, α-olefinsulfonate, alkyl sulfate (fatty alcohol sulfate), alcohol ethoxysulfate, secondary alkanesulfonate, alpha-sulfo fatty acid methyl ester, alkyl- or alkenylsuccinic acid, and soap. Non-ionic surfactants include, without limitation, alcohol ethoxylate, nonylphenol ethoxylate, alkylpolyglycoside, alkyldimethylamineoxide, ethoxylated fatty acid monoethanolamide, fatty acid monoethanolamide, polyhydroxy alkyl fatty acid amide, and N-acyl N-alkyl derivatives of glucosamine ("glucamides").

Builder systems include, without limitation, aluminosilicates, silicates, polycarboxylates and fatty acids, materials such as ethylenediamine tetraacetate, and metal ion sequestrants such as aminopolyphosphonates, particularly ethylenediamine tetramethylene phosphonic acid and diethylene triamine pentamethylenephosphonic acid, which are includeed at a concentration of between about 5% to 80% by weight, preferably between about 5% and about 30% by weight.

Antifoam agents include without limitation silicones (U.S. Patent No. 3,933,672; DC-544 (Dow Corning), which are typically included at a concentration of between about 0.01% and about 1 % by weight

The compositions may also contain soil-suspending agents, soil-releasing agents, optical brighteners, abrasives, and/or bactericides. All of such further components suitable for textile use are well know in the art.

### Process for single solution bleach clean-up and biopoliching

The process of the present invention of one-step combined biopolishing and hydrogen peroxide bleach clean up takes place after hydrogen peroxide bleaching step for treating textile.

The manner in which the aqueous solution containing the enzyme system for BioPolishing and hydrogen peroxide Bleach Clean-up system is contacted with the textile will depend upon whether the processing regime is continuous, discontinuous pad-batch or batch. For example, for continuous or discontinuous pad-batch processing, the aqueous enzyme solution is preferably contained in a saturator bath and is applied continuously to the cotton-containing fabric as it travels through the bath, during which process the cotton-containing fabric typically absorbs the processing liquor at an amount of 0.5-1.5 times its weight. In batch operations, the cotton-containing fabric is exposed to the enzyme solution for a period ranging from about 5 minutes to 24 hours at a liquor-to-fabric ratio of 4:1-50:1.

The aqueous solution or wash liquor typically has a pH of between about 4 and about 11. Preferably, the pH of the treating composition is between about 5 and about 10, preferably between about 5 to about 9, and most preferably about 6 to about 7.

in one embodiment, the single solution method for treating cotton-containing fabric is carried out at a pH below 9, more preferably, below 8, and even more preferably below 7.

The temperature at which the single solution combined hydrogen peroxide Bleach Clean-up and BioPolishing processes are carried out will depend on the process used. In the case of cold pad batch process, the temperature for the single solution combined hydrogen peroxide bleaching clean-up and bio-polishing processes is preferably between about 15°C and about 65°C, and most preferably between about 25°C and about 60°C. For continuous and other batch processes, the temperature for carrying out single solution combined hydrogen peroxide bleaching clean-up and biopolishing processes is preferably between about 35°C and about 75°C, and most preferably between about 45°C and about 65°C.

The cellulase-comprising enzyme system for biopolishing is generally added to the single solution in an amount which is effective to generate enough biopolishing effect of the textile material. The enzyme(s) may preferably be dosed in an amount of 0.01 mg protein/l to 1g protein/l of the total liquor, more preferably from 0,1 mg protein/l to 300mg protein/l, most preferably from 1mg protein/l to 150mg protein/l.

The catalase enzyme for hydrogen peroxide bleach clean up is generally added to the single solution in an amount which is effective to remove hydrogen peroxide. The enzyme(s) may preferably be dosed in an amount about from 0.001mg protein/l to 1g protein/l of the total liquor, preferably 0.01 mg protein/l to 100mg protein/l, most preferably 0.15mg protein/l to about 1 mg protein/l.

It will be understood that the optimum dosage and concentration of the enzymes, bleach compounds, bleach stabilizers, the volume of the aqueous solution or wash liquor, and the pH and temperature will vary, depending on: (i) the nature of the fiber, i.e., crude fiber, yarn, or textile; (ii) the particular enzyme(s) used, and the specific activity of the enzyme; (iii) the conditions of temperature, pH, time, etc., at which the processing occurs; (iv) the presence of other components in the wash liquor; and (v) the type of processing regime used, i.e., continuous, discontinuous pad-batch, or batch. The optimization of the process conditions can be determined using routine experimentation, such as, by establishing a matrix of conditions and testing different points in the matrix. For example, the amount of enzyme, the temperature at which the contacting occurs, and the total time of processing can be varied, after which the resulting cellulosic materials or textile is evaluated for (a) pilling result; and (b) residual H₂O₂ ppm.

Further, according to the present invention, hydrogen peroxide bleach clean up, biopolishing and dyeing steps can be achieved in a single-bath. In one embodiment, (I) adding a single solution comprising catalase and cellulase for one-step hydrogen peroxide bleach clean up and biopolishing, a first incubation is performed for a sufficient time to remove the residual hydrogen peroxide, (ii) the wash liquor is then supplemented with dyestuffs and a second incubation is performed for a sufficient time and under appropriate conditions to achieve effective dyeing. Preferably, after adding catalase and cellulase the incubation time for step (i) is no less than 5 minutes, so most of the hydrogen peroxide has been removed. More preferably, the incubation time for step (i) is no less than 10 minutes, even more preferably no less than 15 minutes and no more than 2 hours. Most preferably, the incubation time for step (i) is 10-20 minutes, so as to remove almost all of the hydrogen peroxide and shorten the whole process time.

It will be understood that before or during step (ii) of dyeing, it further comprise adjusting one or more properties of the composition of the wash liquor, such as ionic strength by adding salt. And the conditions of incubation in dyeing step may also differ with respect to temperature, agitation, pH, time, and the like. The performances of biopolishing with enzymes still continue during incubation of dyeing process, so that the whole process time will be shortened.

Preferably, the dyestuff used in present invention is reactive dye, which works well with cellulase disclosed herein.

The salt added before or after the adding of dyestuff preferably is NaCl or Na₂SO₄ which take control of the uptake of the dyestuff on the fiber. The concentration of salt in the wash liquor depends on the concentration of dyestuff and what kind of dyestuff used. Normally, the higher concentration of dyestuff is required, the higher salt concentration is needed. Typically, the dyestuff in the wash liquor is 0.001-15%, preferably 0.001-9% of the weight of fabric. The concentration of salt (eg. Na₂SO₄) is 10-100 g/l.

The wash liquor in dyeing step preferably has a pH between about 5 and about 8, most preferably between about 6 and about 7.

The temperature at which the subsequent dyeing is carried out may be between about 30°C and about 100°C, preferably between about 40°C and about 90°C, and most preferably between about 40°C and about 80°C.

It will be understood that in most cases, the pH and temperature in bleach clean up and biopolishing step are suitable for the dyeing step, especially for reactive dye. So, there is no need to adjust pH and temperature during dyeing.

The following are intended as non-limiting illustrations of the present invention.

### EXAMPLES

### Materials & Methods

### Enzymes

- Catalase A: Catalase with SEQ ID NO.:3
- Catalase T100 (Genencor international Inc)
- Catalase ASC 200(Mitsubishi)
- Neutral Cellulase B with SEQ ID NO.:2
- Neutral Cellulase A with SEQ ID NO.:1
- Acid Cellulase C : CELLUSOFT L^{™} (NovozymesA/S)
- Primafast Luna RL (Genencor international Inc)
- IndiAge Max L (Genencor international Inc)

### Fabric

- 460-60 bleached Interlock Knits (Testfabrics, Inc.)
- Dyed fabric (Garment bought from Carrfour)

### Dyestuff

- Reactive RED BF-3B (Arugs textile auxiliary CO.LTD)
- Reactive Yellow BF-3R (Arugs textile auxiliary CO.LTD)
- Reactive TQ blue G (Arugs textile auxiliary CO.LTD)

### Buffer

2.716g of potassium dihydrogen phosphate and 0.201g sodium hydroxide are dissolved in 1L de-ionized water. 1g/l NAAC adjust with HAC to pH 5

### Methods:

### Catalase activity (KCIU Assays)

When used according to the present invention the activity of catalase may be measured in KCIU. One CIU will decompose one (µmole of H₂O₂ per minute at pH 7.0, 25°C while the H₂O₂ concentration decreases from 10.3 to 9.2 µmoles per ml reaction mixture.
Catalase catalyzes the first order reaction:

2 H₂O₂→ 2H₂O + O₂

The degradation of hydrogen peroxide is monitored using spectrophotometry at 240 nm. The length of time for a specified decrease in absorbance, at a specified H₂O₂ concentration, is a measure of the catalase activity.

**Reaction**

| | |
|---|---|
| Enzyme concentration | approx.100 CIU/ml |
| Substrate Concentration | 10.3 mM H₂O₂ |
| pH | 7.0 ± 0.05 |
| Buffer | 50 mM phosphate |
| Temperature | 25° C |

| Detection | |
|---|---|
| Wave length | 240 nm |
| Absorbance interval | 0.450-0.400 |
| Time interval | 0.267 - 0.400 minutes (16-24 seconds) |

A folder describing this analytical method in more detail is available upon request to Novozymes A/S, Denmark, which folder is hereby included by reference.

### Cellulase activity (ECU)

Cellulase samples are incubated with a carboxymethyl cellulose (CMC) substrate. Degradation of the substrate leads to a reduction in viscosity, which is measured using a vibrating-spindle viscometer. The reduction in viscosity is pro-portional to the endo-cellulase activity.
Endo-cellulase activity in ECU is measured relative to a Novozymes A/S enzyme standard.

| **Reaction conditions** | |
|---|---|
| Temperature | 40°C |
| pH | 7.5 |
| Substrate concentration | 3.11 %(mN) |
| Incubation time | 30 min |
| Enzyme concentration | 0.097 -0.181 ECU/ml |

A folder describing this analytical method in more detail is available upon request to Novozymes A/S, Denmark, which folder is hereby included by reference.

### Cellulase activity (EGU)

Cellulase samples are incubated with carboxymethyl cellulose (CMC) substrate. Degradation of the substrate leads to a reduction in viscosity, which is measured using a vibrating-spindle viscometer. The reduction in viscosity is proportional to the endo-glucanase activity.

| **Reaction Conditions** | |
|---|---|
| Temperature | 40°C |
| pH | 6.0 |
| Substrate concentration | 3.11 % (w/v) |
| Enzyme concentration | 0.01-0.02 EGU/mL |
| Reaction time | 30 minutes |

A folder describing this analytical method in more detail is available upon request to Novozymes A/S, Denmark, which folder is hereby included by reference.

### Bleach clean up (Peroxide Test-strip)

Peroxidase transfers peroxide oxygen to an organic redox indicator. This produces a blue oxidation product. This peroxide concentration is measured semi-quantitatively by visual comparison of the reaction zone of the analytical test strip with the fields of a colour scale. The hydrogen peroxide is determined by immersing the reaction zone of the analytical test strip in the measurement sample for 1 second. Allow excess liquid to run off via the long edge of strip onto an absorbent paper towel and after 15 sec. determine with which colour field on the label on the colour of the reaction zone coincides most exactly. Read off the corresponding result in mg/l H₂O₂ or if necessary, estimate an intermediate value.

### Pilling test

### Martindale

The principle of the pilling test is that two specimens of a test fabric are rubbed against each other in a continuously changing pattern, which ensures that the surface fibers of the specimens are flexed in every direction. After a predetermined number of rubbing cycles, the wear resistance of the specimens are visually evaluated by comparing to EMPA photo scale standards. There are five grades of the pilling results
Note 5 : No pilling
Note 4 : Slight Pilling
Note 3 : Moderate Pilling
Note 2 : Distinct Pilling
Note 1 : Heavy Pilling
1/2 notes are allowed

The testing method refers to GB/T 4802.2-1997

### The ICI Pilling Tester

The ICI Pilling Tester is the most applicable test to use on most types of fabric, both woven and knitted, and its test method runs in accordance with BS EN ISO 12945-1 ( amongst other standards). The Pilling Tester consists of 2 boxes, each being lined with a metal plate supporting 3.2mm thick buffed-finish cork jointing material. The tester features an automatic counter which stops the machine after any predetermined number of revolutions.

Assessment of the pilling standard is visual, using either the standard photographs or the Rating Scheme. The tested specimens are mounted on card and then rated against the standard photos or against an untested sample.

| Rating | Description | Points to be taken into consideraiton during assessment |
|---|---|---|
| 5 | No change | No visual change |
| 4 | Slight change | Slight surface fuzzing |
| 3 | Moderate change | Test specimen may exhibit moderate fuzzing and/or isolated fully formed pills |
| 2 | Significant change | Distinct fuzzing and/or pilling |
| 1 | Severe change | Dense fuzzing and/or pilling which covers the specimen |

### Protein Content

BCA kit (available from PIERCE) was used for detecting the protein content in the enzyme product.

### Example 1

### Combining Bio-polishing and Bleach clean up into one process conducted with a Laundry-meter

A 100% 460-60 bleached interlock cotton fabric was purchased from Test Fabrics. Fabric swatches were cut to about 5g each.

One buffer at p H 6.5 was used for this study. 2.716g of potassium dihydrogen phosphate and 0 201 g sodium hydroxide were dissolved in 1 L de-ionized water. The process was conducted with a Laundry-meter. The beaker was filled with 100ml buffer and two pieces of pre-cut fabric
1) Main washing: The beaker was filled with 100ml buffer. Some hydrogen peroxide was added to each beaker with 20 steel balls as specified. In the meanwhile, a neutral Cellulase A was dosed to a concentration and catalase A was added in then temperature was raised to 55°C and kept for 60min.
2) Check the residual Hydroperoxide with peroxide strip.
3) Inactivation: After the checking, add the 1g/l Na₂CO₃ in the machine/beaker then raised the temperature to 80°C and run for 10min, drained.
4) Cold rinse: Filled in cold water and rinsed for 10min
5) Spin off the water on the fabrics and tumble dryer.
6) Measure the weight loss and pilling result on the treated fabrics.

The results of the test are shown in Table 1.

**Table 1**

| H₂O₂ At beginning | Catalase A (mg protein /l) | Neutral Cellulase A(mg protein /l) | Residual H₂O₂ after treatment | Pilling result |
|---|---|---|---|---|
| 0 | 0 | 0 | 0 | 1.5-2 |
| 0 | 0 | 7 | 0 | 4-4.5 |
| 100ppm | 0 | 7 | >25ppm | 4-4.5 |
| 0 | 0.34 | 7 | 0 | 4.5 |
| 100ppm | 0.34 | 0 | 0 | 1.5-2 |
| 100ppm | 0.34 | 7 | 0 | 4.5 |
| 350ppm | 0.34 | 4.2 | 0 | 4-4.5 |

As can be seen from the table, the fabrics treated in the combined process of the invention with a combination of catalase and cellulase have less pilling, and no detectable Residual H₂O₂ in the solution.

### Example 2

### Combining Bio-polishing and Bleach clean up into one process conducted with a wascator

A 100% 460-60 bleached interlock cotton fabric was purchased from Test Fabrics. The weight of the fabric swatches was 1 kg.

One buffer at pH 6.5 was used for this study. 2.716g of potassium dihydrogen phosphate and 0.201 g sodium hydroxide were dissolved in 1 L de-ionized water. The process was conducted with a wascator. 1kg fabric was put in the wascator at the beginning of the process.
1) Main washing: The wascator was filled with 10L water. The temperature was raised to 55°C. In the mean while, 36g of K₂HPO₄ and 14.5g KH₂PO₄ were added in to adjust the pH to 6.5. Some hydrogen peroxide was added to the wascator as specified. A neutral Cellulase was dosed to a concentration and catalase was added in and kept for 60min.
2) Check the residual Hydroperoxide with peroxide strip after 20min.
3) Inactivation: After main washing, drained and re-fill the wascator and added the 1 g/l Na₂CO₃ in the wascator then raised the temperature to 80°C and run for 10min, drained.
4) Cold rinse: Filled in cold water and rinsed for 10min
5) Spin off the water on the fabrics and tumble dryer.
6) Measure the pilling result on the treated fabrics.

The result of the testing are shown in Table 2

**Table 2**

| H₂O₂ At beginning | Catalase A (mg protein /l) | Neutral Cellulase A (mg protein /l) | Residual H₂O₂ after treatment for 20min | Pilling result |
|---|---|---|---|---|
| 100ppm | 0.34 | 7 | 0 | 4.5 |

As can be seen from the table, the fabrics treated in the combined process of the invention with a combination of catalase A and neutral cellulase A have less pilling, and no detectable Residual H₂O₂ in the solution.

### Example 3

Combining Bio-polishing and Bleach clean up into one process conducted on dyed fabric with a Laundrymeter

A 100% dyed cotton fabric was purchased. Fabric swatches were cut to about 10g each.

One buffer at pH 6.5 was used for this study. 2.716g of potassium dihydrogen phosphate and 0.201 g sodium hydroxide were dissolved in 1 L de-ionized water. The process was conducted with a Laundry-meter. The beaker was filled with 100ml buffer and one piece of pre-cut fabric.
1) Main washing: The beaker was filled with 100ml buffer. Some hydrogen peroxide was added to each beaker with 20 steel balls as specified. In the meanwhile, a neutral Cellulase A was dosed to a concentration and catalase A was added in then temperature was raised to 55°C and kept for 20min.
2) Check the residual Hydroperoxide with peroxide strip.
3) Inactivation: After the checking, add the 1g/l Na₂CO₃ in the machine/beaker then raised the temperature to 80°C and run for 10min, drained.
4) Cold rinse: Filled in cold water and rinsed for 10min
5) Spin off the water on the fabrics and tumble dryer.
6) Measure pilling result on the treated fabrics.

The results of the test are shown in Table 3.

**Table 3**

| H₂O₂ At beginning | Catalase A (mg protein /l) | Neutral Cellulase A (mg protein /l) | Residual H₂O₂ after treatment | Pilling result |
|---|---|---|---|---|
| 0 | 0 | 0 | 0 | 2-2.5 |
| 350ppm | 0 | 0 | >25ppm | 2-2.5 |
| 0 | 0 | 4.2 | 0 | 4 |
| 350ppm | 0 | 4.2 | >25ppm | 3.85 |
| 350ppm | 0.34 | 4.2 | 0 | 4 |
| 0 | 0 | 21 | 0 | 4.5-5 |
| 350ppm | 0 | 21 | >25ppm | 4.5-5 |
| 350ppm | 0.34 | 21 | 0 | 4.5-5 |

As can be seen from the above table, the fabrics treated in the combined process of the invention with a combination of catalase and cellulase have less pilling, and no detectable residual H₂O₂ in the solution. The present invention shows more advantageous effect than those using catalase or cellulase alone.

### Example 4

### Combining Bio-polishing and Bleach clean up into one process with an acid cellulase

A 100% 460-60 bleached interlock cotton fabric was purchased from Test Fabrics. Fabric swatches were cut to about 5g each.

One buffer at pH 5 was used for this study. 1g of sodium acetate were dissolved in 1L de-ionized water and adjust the pH to 5 with acetic acid. The process was conducted with a Laundry-meter. The beaker was filled with 100ml buffer and two pieces of pre-cut fabric.
1) Main washing: The beaker was filled with 100ml buffer. Some hydrogen peroxide was added to each beaker with 20 steel balls as specified. In the meanwhile, an acid Cellulase C was dosed to a concentration and catalase A was added in then temperature was raised to 55°C and kept for 60min.
2) Check the residual Hydroperoxide with peroxide strip.
3) Inactivation: After the checking, add the 1g/l Na₂CO₃ in the machine/beaker then raised the temperature to 80°C and run for 10min, drained.
4) Cold rinse: Filled in cold water and rinsed for 10min
5) Spin off the water on the fabrics and tumble dryer.
6) Measure the weight loss and pilling result on the treated fabrics.

The results of the test are shown in Table 4.

**Table 4**

| H₂O₂ At beginning | Catalase A (mg protein /l) | Acid Cellulase C (mg protein /l) | Residual H₂O₂ after treatment | Pilling result |
|---|---|---|---|---|
| 0 | 0 | 0 | 0 | 1.5-2 |
| 0 | 0 | 120 | 0 | 4-4.5 |
| 100ppm | 0.34 | 120 | 0 | 3.5-4 |

### Example 5

### Combining Bio-polishing and Bleach clean up into one process with Primafast luna RL

The procedures were the same as described in Example 4 whilst the cellulase used was Primafast luna RL and the catalse used was Catalase A or Catalase T100, ASC 200 individually. The result of the test was shown in Table 5.

### Example 6

### Combining Bio-polishing and Bleach clean up into one process with IndiAge Max L

The procedures were the same as described in Example 4 whilst the cellulase used was Primafast luna RL and the catalse used was Catalase A or Catalase T100, ASC 200 individually. The result of the test was shown in Table 5.

### Example 7

### Combining Bio-polishing and Bleach clean up into one process with Neutral cellulase A

The procedures were the same as described in Example 1 whilst the Catalase used was Catalase T 100 or ASC 200. The result of the test was shown in Table 5.

### Example 8

### Combining Bio-polishing and Bleach clean up into one process with Acid cellulase C

The procedures were the same as described in Example 4 whilst the Catalase used was Catalase T 100 or ASC 200. The result of the test was shown in Table 5

**Table 5**

| H₂O₂ At beginning | Cellulase | Dosage (mg protein /l) | Catalase | Dosage (mg protein /l) | Pilling result | Residual H₂O₂ after treatment |
|---|---|---|---|---|---|---|
| / | Primafast luna RL | 41.88 | / | / | 3.85 | 0 |
| 350ppm | Primafast luna RL | 41.88 | Catalase A | 0.34 | 4-4.5 | 0 |
| 350ppm | Primafast luna RL | 41.88 | Catalase T 100 | 1 | 3.85 | 0 |
| 350ppm | Primafast luna RL | 41.88 | ASC 200 | 0.58 | 3.85 | 0 |
| / | IndiAge Max L | 69 | / | / | 4.5-5 | 0 |
| 350ppm | IndiAge Max L | 69 | Catalase A | 0.34 | 4-4.5 | 0 |
| 350ppm | IndiAge Max L | 69 | Catalase T 100 | 1 | 4-4.5 | 0 |
| 350ppm | IndiAge Max L | 69 | ASC 200 | 0.58 | 4-4.5 | 0 |
| / | Neutral cellulase A | 1.75 | / | / | 4-4.5 | 0 |
| 350ppm | Neutral cellulase A | 1.75 | Catalase T 100 | 1 | 3.5-4 | 0 |
| 350ppm | Neutral cellulase A | 1.75 | ASC 200 | 0.58 | 4-4.5 | 0 |
| / | Acid cellulase C | 120 | / | / | 3.5-4 | 0 |
| 350ppm | Acid cellulase C | 120 | Catalase T 100 | 1 | 3.5-4 | 0 |
| 350ppm | Acid cellulase C | 120 | ASC 200 | 0.58 | 3.5-4 | 0 |

### Example 9

### Combining Biopolishing and Bleach clean up into one process conducted with a Laundry-meter at lower temp

A 100% 460-60 bleached interlock cotton fabric was purchased from Test Fabrics. Fabric swatches were cut to about 5g each.

One buffer at pH 6.5 was used for this study. 2.716g of potassium dihydrogen phosphate and 0.201g sodium hydroxide were dissolved in 1L de-ionized water. The process was conducted with a Laundry-meter. The beaker was filled with 100ml buffer and two pieces of pre-cut fabric.
1) Main washing: The beaker was filled with 100ml buffer. Some hydrogen peroxide was added to each beaker with 20 steel balls as specified. In the meanwhile, a neutral Cellulase B was dosed to a concentration and catalase A was added in then temperature was raised to 30°C and kept for 60min.
2) Check the residual Hydroperoxide with peroxide strip.
3) Inactivation: After the checking, add the 1g/l Na₂CO₃ in the machine/beaker then raised the temperature to 80°C and run for 10min, drained.
4) Cold rinse: Filled in cold water and rinsed for 10min
5) Spin off the water on the fabrics and tumble dryer.
6) Measure the weight loss and pilling result on the treated fabrics.

**Table 6**

| H₂O₂ At beginning | Cellulase | Dosage (mg protein /l) | Catalase | Dosage (mg protein /l) | Pilling result | Residual H₂O₂ after treatment |
|---|---|---|---|---|---|---|
| / | / | / | / | / | 1.5-2 | 0 |
| / | Neutral cellualse B | 6 | / | / | 4 | 0 |
| 350ppm | Neutral cellualse B | 6 | Catalase A | 0.34 | 4 | 0 |
| / | Neutral cellualse A | 12 | / | / | 4.-4.5 | 0 |
| 350ppm | Neutral cellualse B | | | | | |
| 350ppm | Neutral cellualse B | | | | | |

### Example 10 (comparative example)

### Combining Biopolishing and Bleach clean up with reducing agent into one process conducted with a Laundry-meter

A 100% 460-60 bleached interlock cotton fabric was purchased from Test Fabrics. Fabric swatches were cut to about 5g each.

One buffer at p H 6.5 was used for this study. 2.716g of potassium dihydrogen phosphate and 0.201g sodium hydroxide were dissolved in 1L de-ionized water. The process was conducted with a Laundry-meter. The beaker was filled with 100ml buffer and two pieces of pre-cut fabric.
1) Main washing: The beaker was filled with 100ml buffer. Some hydrogen peroxide was added to each beaker with 20 steel balls as specified. In the meanwhile, a neutral Cellulase B was dosed to a concentration and reducing agent
   (sodium hyposulphate: abbreviated as Hypo) was added in then temperature was raised to 55°C and kept for 60min.
2) Check the residual Hydroperoxide with peroxide strip.
3) Inactivation: After the checking, add the 1 g/l Na2CO3 in the machine/beaker then raised the temperature to 80°C and run for 10min, drained.
4) Cold rinse: Filled in cold water and rinsed for 10min
5) Spin off the water on the fabrics and tumble dryer.
6) Measure the weight loss and pilling result on the treated fabrics.

The results of the test are shown in Table 7.

**Table 7**

| H₂O₂ At beginning | Enzyme | Dosage (mg protein /l) | Reducing agent | Weight loss% | Residual H₂O₂ | Pilling result |
|---|---|---|---|---|---|---|
| / | | blank | / | -0.6 | 0 | 1.625 |
| H₂O₂ 350PPM | Neutral Cellulase B | 3 | / | 2.9 | >25ppm | 4.875 |
| H₂O₂ 350PPM | Neutral Cellulase B | 6 | / | 4.3 | >25ppm | 5 |
| H₂O₂ 350PPM | Neutral Cellulase B | 12 | / | 6.5 | >25ppm | 5 |
| H₂O₂ 100PPM | Neutral Cellulase B | 3 | 1g/l Hypo | 3.4 | 0 | 5 |
| H₂O₂ 100PPM | Neutral Cellulase B | 6 | 1g/l Hypo | 4.9 | 0 | 5 |
| H₂O₂ 100PPM | Neutral Cellulase B | 12 | 1g/l Hypo | 6.6 | 0 | 5 |

### Example 11

### One bath Biopolishing, Bleach clean up and dyeing conducted with a Laundry-meter

A 100% 460-60 bleached interlock cotton fabric was purchased from Test Fabrics. Fabric swatches were cut to about 5g each.

**Table 8: Dyeing recipe**

| Sample No | Sample 1 | Sample 2 | Sample 3 | Sample 4 | Sample 5 | Sample 6 |
|---|---|---|---|---|---|---|
| Chemicals used | Dosage | | | | | |
| H₂O₂ | 300ppm | / | 300ppm | / | 300ppm | / |
| Neutral Cellulase A (protein/L) | 7mg/L | / | 7mg/L | / | 7mg/L | / |
| Catalase A (protein/L) | 0.34mg/l | | 0.34mg/l | | 0.34mg/l | |
| Reactive RED BF-3B | 1 % owf | 1 % owf | 2 % owf | 2% owf | 3% owf | 3% owf |
| Reactive Yellow BF-3R | 1 % owf | 1 % owf | 2 % owf | 2% owf | 3% owf | 3% owf |
| Reactive TQ Blue G | 1 % owf | 1 % owf | 2% owf | 2 % owf | 3% owf | 3% owf |
| Na₂SO₄ | 40g/l | 40g/l | 60g/l | 60g/l | 80g/l | 80g/l |
| Na₂CO₃ | 15g/l | 15g/l | 20g/l | 20g/l | 20g/l | 20g/l |
| Owf: of the weight of fabric | | | | | | |

A water solution at pH 6.5 was used for this study. Acetic acid was dissolved in 1L de-ionized water to adjust pH to 6.5. The process was conducted with a Laundry-meter. The beaker was filled with 100ml solution and two pieces of pre-cut fabric.
1) Single-bath bleach clean up, bio-polishing and dyeing process:
   The beaker was filled with 100ml water solution at pH 6.5 adjusted by acetic acid. Some hydrogen peroxide was added to each beaker with 20 steel balls as specified. In the meanwhile, a neutral Cellulase A was dosed to a concentration and catalase A was added, following that certain amount of sodium sulfate was added in, then temperature was raised to 60°C and kept for 15min. Check the residual Hydroperoxide with peroxide strip.
2) Adding dyestuff and sodium carbonate: After the checking, add certain concentration of dyestuffs (three kinds of dyestuff from Argus) in the machine/beaker and run for 45min, then dosed certain amount sodium carbonate and run for 45min/60min. Drained.
3) Soaping: two steps of soaping process were conducted following the dyeing process. Filled in cold water and added 1g/l soaping agent Dekol SNS(BASF) and raised temperature to 90°C for 10min. Drained. Filled in cold water and added 1g/l soaping agent Dekol SNS (BASF) and raised temperature to 90°C for 10min. Drained.
4) Hot rinse: Filled in cold water and raised temperature to 70°C for 15min.
5) Cold rinse: Filled in cold water and rinsed for 10min.
6) Spin off the water on the fabrics and tumble dryer.
7) Measure the pilling result on the treated fabrics.

The results of the test are shown in Table 9.

**Table 9**

| Sample No | Sample 1 | Sample 2 | Sample 3 | Sample 4 | Sample 5 | Sample 6 |
|---|---|---|---|---|---|---|
| Residual H₂O₂ before adding dyestuff | 0 | n/a | 0 | n/a | 0 | n/a |
| Pilling | 4.5 | 1.5 | 4.25 | 1.5 | 4.25 | 1.5 |

As can be seen from the above table, the fabrics treated in the one bath bleach clean up, bio-polishing and dyeing process of the invention with a combination of catalase and cellulase have less pilling and largely shorten the process time compared to the separated process.

## Claims

1. A one-step textile treatment process for combined biopolishing and hydrogen peroxide bleach clean up comprising the step of treating the textile within a single aqueous enzyme solution containing (i) a catalase and (ii) an enzyme system comprising cellulase for bio-polishing, wherein the combined biopolishing and hydrogen peroxide bleach clean up process takes place after a hydrogen peroxide bleaching step for treating the textile.

2. The process of claim 1, wherein the textile comprises cellulose-containing or cellulosic fabrics.

3. The process of any of claims 1 or 2, wherein the enzyme system for biopolishing comprises an endoglucanase.

4. The process of claim 1, wherein the cellulase is a family 45 cellulase, preferably, the family 45 cellulase is selected from the group consisting of a polypeptide comprising the sequence of SEQ ID NO: 1 or SEQ ID NO.2, or a variant thereof which has at least 70% identity to SEQ ID NO: 1 or SEQ ID NO: 2.

5. The process of any of the preceding claims, wherein the catalase is derived from bacteria such as *Bacillus, Pseudomonas or Streptomyces strain;* yeast such as *Candida, Kluyveromyces, Pichia, Saccharomyces, Yarrowia or Schizosaccharomyces*; fungal such as *Acremonium, Scytalidium, Aspergillus, Coprinus, Aureobasidium, Bjerkandera, Humicola, Ceriporiopsis, Coriolus, Cryptococcus, Filibasidium, Fusarium, Magnaporthe, Mucor, Myceliphthora, Neocallimastix, Neurospora, Paecilomyces, Penicillium, Phanerochaete, Phlebia, Piromyces, Pleurotus, Schizophyllum, Talaromyces, Thermoascus, Thielavia, Tolypocladium, Trametes, Micrococcus or Trichoderma strain*; or animal such as pig liver, beef liver.

6. The process of any of the preceding claims, wherein the pH of the solution is between about 4 and about 12, preferably between about 6 and about 8.

7. The process of any of the preceding claims, wherein the process is carried out at a temperature between 20 degree Celsius to 80 degree Celsius, preferably between 50 degree Celsius to 60 degree Celsius.

8. A method according to any preceding claim, wherein the aqueous enzyme solution is supplemented with one or more dyestuffs and the method comprises incubating the textile for a sufficient time to achieve dyed textile.

## Patentansprüche

1. Einstufiger Textilbehandlungsvorgang für kombiniertes Biopolishing und Reinigen mit Wasserstoffperoxidbleiche, umfassend den Schritt des Behandelns der Textilie innerhalb einer einzigen wässrigen Enzymlösung, die Folgendes enthält: (i) eine Catalase und (ii) ein Enzymsystem, das Cellulase für Biopolishing umfasst, wobei der Vorgang für kombiniertes Biopolishing und Reinigen mit Wasserstoffperoxidbleiche nach einem Wasserstoffperoxidbleichschritt zum Behandeln der Textilie stattfindet.

2. Vorgang nach Anspruch 1, wobei die Textilie cellulosehaltiges oder Cellulose-Gewebe enthält.

3. Vorgang nach einem der Ansprüche 1 und 2, wobei das Enzymsystem für Biopolishing eine Endoglucanase umfasst.

4. Vorgang nach Anspruch 1, wobei die Cellulase eine Familie-45-Cellulase ist, wobei die Familie-45-Cellulase vorzugsweise ausgewählt ist aus der Gruppe bestehend aus einem Polypeptid, umfassend die Sequenz von SEQ ID NO: 1 oder SEQ ID NO.2, oder eine Variante davon mit einer wenigstens 70%igen Identität mit SEQ ID NO: 1 oder SEQ ID NO: 2.

5. Vorgang nach einem der vorhergehenden Ansprüche, wobei die Catalase abgeleitet ist aus Bakterien wie etwa vom Stamm *Bacillus, Pseudomonas* oder *Streptomyces;* aus Hefepilzen wie etwa *Candida, Kluyveromyces, Pichia, Saccharomyces, Yarrowia* oder *Schizosaccharomyces;* aus Pilzen wie etwa vom Stamm *Acremonium, Scytalidium, Aspergillus, Coprinus, Aureobasidium, Bjerkandera, Humicola, Ceriporiopsis, Coriolus, Cryptococcus, Filibasidium, Fusarium, Magnaporthe, Mucor, Myceliphthora, Neocallimastix, Neurospora, Paecilomyces, Penicillium, Phanerochaete, Phlebia, Piromyces, Pleurotus, Schizophyllum, Talaromyces, Thermoascus, Thielavia, Tolypocladium, Trametes, Micrococcus* oder *Trichoderma;* oder aus Tieren, wie etwa aus Schweineleber oder Rinderleber.

6. Vorgang nach einem der vorhergehenden Ansprüche, wobei der pH-Wert der Lösung zwischen etwa 4 und etwa 12, vorzugsweise zwischen etwa 6 und etwa 8 liegt.

7. Vorgang nach einem der vorhergehenden Ansprüche, wobei der Vorgang bei einer Temperatur von zwischen 20 °C und 80 °C, vorzugsweise zwischen 50 °C und 60 °C ausgeführt wird.

8. Verfahren nach einem der vorhergehenden Ansprüche, wobei die wässrige Enzymlösung mit einem oder mehreren Färbemitteln ergänzt ist und das Verfahren das Inkubieren der Textilie ausreichend lange zum Erzielen einer gefärbten Textilie umfasst.

## Revendications

1. Procédé de traitement textile à une étape pour un biopolissage et un nettoyage de blanchiment à l'eau oxygénée combinés comprenant l'étape de traitement du textile au sein d'une solution aqueuse unique contenant (i) une catalase et (ii) un système d'enzyme comprenant une cellulase pour le biopolissage, dans lequel le procédé de biopolissage et de nettoyage de blanchiment à l'eau oxygénée combinés a lieu après une étape de blanchiment à l'eau oxygénée destinée à traiter le textile.

2. Procédé selon la revendication 1, dans lequel le textile comprend des tissus cellulosiques ou contenant de la cellulose.

3. Procédé selon l'une quelconque des revendications 1 ou 2, dans lequel le système d'enzyme pour le biopolissage comprend une endoglucanase.

4. Procédé selon la revendication 1, dans lequel la cellulase est une cellulase de la famille 45, de préférence la cellulase de la famille 45 est choisie dans le groupe consistant en un polypeptide comprenant la séquence SEQ ID n° : 1 ou SEQ ID n° : 2, ou un variant de celle-ci qui a au moins 70 % d'identité avec SEQ ID n° : 1 ou SEQ ID n° : 2.

5. Procédé selon l'une quelconque des revendications précédentes, dans lequel la catalase est dérivée de bactéries telles qu'une souche de *Bacillus, Pseudomonas* ou *Streptomyces ;* une levure telle que *Candida, Kluyveromyces, Pichia, Saccharomyces, Yarrowia,* ou *Schizosaccharomyces ;* un champignon tel qu'une souche de *Acremonium, Scytalidium, Aspergillus, Coprinus, Aureobasidium, Bjerkandera, Humicola, Ceriporiopsis, Coriolus, Cryptococcus, Filibasidium, Fusarium, Magnaporte, Mucor, Myceliphthora, Neocallimastix, Neurospora, Paecilomyces, Penicillium, Phanerochaete, Phlebia, Piromyces, Pleurotus, Schizophyllum, Talaromyces, Thermoascus, Thielavia, Tolypocladium, Trametes, Micrococcus* ou *Trichoderma ;* ou d'un animal tel que le foie de cochon ou le foie de oeuf.

6. Procédé selon l'une quelconque des revendications précédentes, dans lequel le pH de la solution est compris entre environ 4 et environ 12, de préférence entre environ 6 et environ 8.

7. Procédé selon l'une quelconque des revendications précédentes, dans lequel le procédé est réalisé à une température comprise entre 20 degrés Celsius et 80 degrés Celsius, de préférence entre 50 degrés Celsius et 60 degrés Celsius.

8. Procédé selon une quelconque revendication précédente, dans lequel la solution aqueuse d'enzyme est supplémentée par une ou plusieurs teintures et le procédé comprend l'incubation du textile pendant un temps suffisant pour parvenir à un textile teinté.


### SEQUENCE LISTING

<110> Novozymes A/S
<120> A Process for Combined BioPolishing and Bleach clean-up
<130> 11222.204-WO
<160> 3
<170> PatentIn version 3.4
<210> 1
   <211> 299
   <212> PRT
   <213> Thielavia terrestis
<400> 1
   Met Arg Ser Thr Pro Val Leu Arg Thr Thr Leu Ala Ala Ala Leu Pro
   1 5 10 15
   Leu Val Ala Ser Ala Ala Ser Gly Ser Gly Gln Ser Thr Arg Tyr Trp
   20 25 30
   Asp Cys Cys Lys Pro Ser Cys Ala Trp Pro Gly Lys Ala Ala Val Ser
   35 40 45
   Gln Pro Val Tyr Ala Cys Asp Ala Asn Phe Gln Arg Leu Ser Asp Phe
   50 55 60
   Asn Val Gln Ser Gly Cys Asn Gly Gly Ser Ala Tyr Ser Cys Ala Asp
   65 70 75 80
   Gln Thr Pro Trp Ala Val Asn Asp Asn Leu Ala Tyr Gly Phe Ala Ala
   85 90 95
   Thr Ser Ile Ala Gly Gly Ser Glu Ser Ser Trp Cys Cys Ala Cys Tyr
   100 105 110
   Ala Leu Thr Phe Thr Ser Gly Pro Val Ala Gly Lys Thr Met Val Val
   115 120 125
   Gln Ser Thr Ser Thr Gly Gly Asp Leu Gly Ser Asn Gln Phe Asp Ile
   130 135 140
   Ala Met Pro Gly Gly Gly Val Gly Ile Phe Asn Gly Cys Ser Ser Gln
   145 150 155 160
   Phe Gly Gly Leu Pro Gly Ala Gln Tyr Gly Gly Ile Ser Ser Arg Asp
   165 170 175
   Gln Cys Asp Ser Phe Pro Ala Pro Leu Lys Pro Gly Cys Gln Trp Arg
   180 185 190
   Phe Asp Trp Phe Gln Asn Ala Asp Asn Pro Thr Phe Thr Phe Gln Gln
   195 200 205
   Val Gln Cys Pro Ala Glu Ile Val Ala Arg Ser Gly Cys Lys Arg Asn
   210 215 220
   Asp Asp Ser Ser Phe Pro Val Phe Thr Pro Pro Ser Gly Gly Asn Gly
   225 230 235 240
   Gly Thr Gly Thr Pro Thr Ser Thr Ala Pro Gly Ser Gly Gln Thr Ser
   245 250 255
   Pro Gly Gly Gly Ser Gly Cys Thr Ser Gln Lys Trp Ala Gln Cys Gly
   260 265 270
   Gly Ile Gly Phe Ser Gly Cys Thr Thr Cys Val Ser Gly Thr Thr Cys
   275 280 285
   Gln Lys Leu Asn Asp Tyr Tyr Ser Gln Cys Leu
   290 295
<210> 2
   <211> 305
   <212> PRT
   <213> Humicola insolens
<400> 2
   Met Arg Ser Ser Pro Leu Leu Arg Ser Ala Val Val Ala Ala Leu Pro
   1 5 10 15
   Val Leu Ala Leu Ala Ala Asp Gly Arg Ser Thr Arg Tyr Trp Asp Cys
   20 25 30
   Cys Lys Pro Ser Cys Gly Trp Ala Lys Lys Ala Pro Val Asn Gln Pro
   35 40 45
   Val Phe Ser Cys Asn Ala Asn Phe Gln Arg Ile Thr Asp Phe Asp Ala
   50 55 60
   Lys Ser Gly Cys Glu Pro Gly Gly Val Ala Tyr Ser Cys Ala Asp Gln
   65 70 75 80
   Thr Pro Trp Ala Val Asn Asp Asp Phe Ala Leu Gly Phe Ala Ala Thr
   85 90 95
   Ser Ile Ala Gly Ser Asn Glu Ala Gly Trp Cys Cys Ala Cys Tyr Glu
   100 105 110
   Leu Thr Phe Thr Ser Gly Pro Val Ala Gly Lys Lys Met Val Val Gln
   115 120 125
   Ser Thr Ser Thr Gly Gly Asp Leu Gly Ser Asn His Phe Asp Leu Asn
   130 135 140
   Ile Pro Gly Gly Gly Val Gly Ile Phe Asp Gly Cys Thr Pro Gln Phe
   145 150 155 160
   Gly Gly Leu Pro Gly Gln Arg Tyr Gly Gly Ile Ser Ser Arg Asn Glu
   165 170 175
   Cys Asp Arg Phe Pro Asp Ala Leu Lys Pro Gly Cys Tyr Trp Arg Phe
   180 185 190
   Asp Trp Phe Lys Asn Ala Asp Asn Pro Ser Phe Ser Phe Arg Gln Val
   195 200 205
   Gln Cys Pro Ala Glu Leu Val Ala Arg Thr Gly Cys Arg Arg Asn Asp
   210 215 220
   Asp Gly Asn Phe Pro Ala Val Gln Ile Pro Ser Ser Ser Thr Ser Ser
   225 230 235 240
   Pro Val Asn Gln Pro Thr Ser Thr Ser Thr Thr Ser Thr Ser Thr Thr
   245 250 255
   Ser Ser Pro Pro Val Gln Pro Thr Thr Pro Ser Gly Cys Thr Ala Glu
   260 265 270
   Arg Trp Ala Gln Cys Gly Gly Asn Gly Trp Ser Gly Cys Thr Thr Cys
   275 280 285
   Val Ala Gly Ser Thr Cys Thr Lys Ile Asn Asp Trp Tyr His Gln Cys
   290 295 300
   Leu
   305
<210> 3
   <211> 717
   <212> PRT
   <213> Scytalidium thermophilum
<400> 3
   Met Asn Arg Val Thr Asn Leu Leu Ala Trp Ala Gly Ala Ile Gly Leu
   1               5                   10                  15
   Ala Gln Ala Thr Cys Pro Phe Ala Asp Pro Ala Ala Leu Tyr Ser Arg
               20                  25                  30
   Gln Asp Thr Thr Ser Gly Gln Ser Pro Leu Ala Ala Tyr Glu Val Asp
           35                  40                  45
   Asp Ser Thr Gly Tyr Leu Thr Ser Asp Val Gly Gly Pro Ile Gln Asp
       50                  55                  60
   Gln Thr Ser Leu Lys Ala Gly Ile Arg Gly Pro Thr Leu Leu Glu Asp
   65                  70                  75                  80
   Phe Met Phe Arg Gln Lys Ile Gln His Phe Asp His Glu Arg Val Pro
   85 90 95
   Glu Arg Ala Val His Ala Arg Gly Ala Gly Ala His Gly Thr Phe Thr
   100 105 110
   Ser Tyr Ala Asp Trp Ser Asn Ile Thr Ala Ala Ser Phe Leu Asn Ala
   115 120 125
   Thr Gly Lys Gln Thr Pro Val Phe Val Arg Phe Ser Thr Val Ala Gly
   130 135 140
   Ser Arg Gly Ser Ala Asp Thr Ala Arg Asp Val His Gly Phe Ala Thr
   145 150 155 160
   Arg Phe Tyr Thr Asp Glu Gly Asn Phe Asp Ile Val Gly Asn Asn Ile
   165 170 175
   Pro Val Phe Phe Ile Gln Asp Ala Ile Gln Phe Pro Asp Leu Ile His
   180 185 190
   Ser Val Lys Pro Arg Pro Asp Asn Glu Ile Pro Gln Ala Ala Thr Ala
   195 200 205
   His Asp Ser Ala Trp Asp Phe Phe Ser Gln Gln Pro Ser Thr Met His
   210 215 220
   Thr Leu Phe Trp Ala Met Ser Gly His Gly Ile Pro Arg Ser Tyr Arg
   225 230 235 240
   His Met Asp Gly Phe Gly Val His Thr Phe Arg Phe Val Lys Asp Asp
   245 250 255
   Gly Ser Ser Lys Leu Ile Lys Trp His Phe Lys Ser Arg Gln Gly Lys
   260 265 270
   Ala Ser Leu Val Trp Glu Glu Ala Gln Val Leu Ser Gly Lys Asn Ala
   275 280 285
   Asp Phe His Arg Gln Asp Leu Trp Asp Ala Ile Glu Ser Gly Asn Gly
   290 295 300
   Pro Glu Trp Asp Val Cys Val Gln Ile Val Asp Glu Ser Gln Ala Gln
   305 310 315 320
   Ala Phe Gly Phe Asp Leu Leu Asp Pro Thr Lys Ile Ile Pro Glu Glu
   325 330 335
   Tyr Ala Pro Leu Thr Lys Leu Gly Leu Leu Lys Leu Asp Arg Asn Pro
   340 345 350
   Thr Asn Tyr Phe Ala Glu Thr Glu Gln Val Met Phe Gln Pro Gly His
   355 360 365
   Ile Val Arg Gly Ile Asp Phe Thr Glu Asp Pro Leu Leu Gln Gly Arg
   370 375 380
   Leu Phe Ser Tyr Leu Asp Thr Gln Leu Asn Arg Asn Gly Gly Pro Asn
   385 390 395 400
   Phe Glu Gln Leu Pro Ile Asn Met Pro Arg Val Pro Ile His Asn Asn
   405 410 415
   Asn Arg Asp Gly Ala Gly Gln Met Phe Ile His Arg Asn Lys Tyr Pro
   420 425 430
   Tyr Thr Pro Asn Thr Leu Asn Ser Gly Tyr Pro Arg Gln Ala Asn Gln
   435 440 445
   Asn Ala Gly Arg Gly Phe Phe Thr Ala Pro Gly Arg Thr Ala Ser Gly
   450 455 460
   Ala Leu Val Arg Glu Val Ser Pro Thr Phe Asn Asp His Trp Ser Gln
   465 470 475 480
   Pro Arg Leu Phe Phe Asn Ser Leu Thr Pro Val Glu Gln Gln Phe Leu
   485 490 495
   Val Asn Ala Met Arg Phe Glu Ile Ser Leu Val Lys Ser Glu Glu Val
   500 505 510
   Lys Lys Asn Val Leu Thr Gln Leu Asn Arg Val Ser His Asp Val Ala
   515 520 525
   Val Arg Val Ala Ala Ala Ile Gly Leu Gly Ala Pro Asp Ala Asp Asp
   530 535 540
   Thr Tyr Tyr His Asn Asn Lys Thr Ala Gly Val Ser Ile Val Gly Ser
   545 550 555 560
   Gly Pro Leu Pro Thr Ile Lys Thr Leu Arg Val Gly Ile Leu Ala Thr
   565 570 575
   Thr Ser Glu Ser Ser Ala Leu Asp Gln Ala Ala Gln Leu Arg Thr Arg
   580 585 590
   Leu Glu Lys Asp Gly Leu Val Val Thr Val Val Ala Glu Thr Leu Arg
   595 600 605
   Glu Gly Val Asp Gln Thr Tyr Ser Thr Ala Asp Ala Thr Gly Phe Asp
   610 615 620
   Gly Val Val Val Val Asp Gly Ala Ala Ala Leu Phe Ala Ser Thr Ala
   625                 630                 635                 640
   Ser Ser Pro Leu Phe Pro Thr Gly Arg Pro Leu Gln Ile Phe Val Asp
                   645                 650                 655
   Ala Tyr Arg Trp Gly Lys Pro Val Gly Val Cys Gly Gly Lys Ser Ser
               660                 665                 670
   Glu Val Leu Asp Ala Ala Asp Val Pro Glu Asp Gly Asp Gly Val Tyr
           675                 680                 685
   Ser Glu Glu Ser Val Asp Met Phe Val Glu Glu Phe Glu Lys Gly Leu
       690                 695                 700
   Ala Thr Phe Arg Phe Thr Asp Arg Phe Ala Leu Asp Ser
   705                 710                 715